# EUROPÄISCHE PATENTSCHRIFT

(11) **EP 1 192 121 B1**
(45) Veröffentlichungstag und Bekanntmachung des Hinweises auf die Patenterteilung: **15.02.2006**
(21) Anmeldenummer: 00935173.5
(22) Anmeldetag: 06.06.2000
(51) Int. Cl.: C07C 205/58, C07C 205/12, C07C 205/26, C07C 201/12

(54) **VERFAHREN ZUR HERSTELLUNG VON 2-HALO-6-NITROBENZOESÄUREN**
METHOD FOR PRODUCING 2-HALO-6-NITROBENZOIC ACIDS
PROCEDE DE PRODUCTION D'ACIDES 2-HALO-6-NITROBENZOIQUES

(30) Priorität: 16.06.1999 DE 19927408
(43) Veröffentlichungstag der Anmeldung: 03.04.2002
(73) Patentinhaber: LANXESS Deutschland GmbH, 51369 Leverkusen (DE)
(72) Erfinder: LANGER, Reinhard, D-47918 Tönisvorst (DE); KLAUSENER, Alexander, D-50259 Pulheim (DE)
(86) Internationale Anmeldenummer: PCT/EP2000/005168
(87) Internationale Veröffentlichungsnummer: WO 2000/076951

(56) Entgegenhaltungen:
- DE-A- 2 708 115
- DE-A- 3 409 244
- US-A- 2 387 341
- US-A- 2 407 182
- US-A- 2 429 493

## Beschreibung

Die vorliegende Erfindung betrifft ein Verfahren zur Herstellung von 2-Halo-6-nitrobenzoesäuren durch Oxidation von 2-Halo-6-nitro-benzylalkoholen und/oder deren Ester und/oder Ether mit Salpetersäure und den Einsatz dieses Verfahrens als Schlüsselschritt bei der Herstellung von 2-Halo-6-nitrobenzoesäuren ausgehend von 2-Halo-6-nitrotoluolen.

Zur Herstellung von 2-Halo-6-nitrobenzoesäuren sind nur wenige Verfahrensweisen Stand der Technik:

B.D. Andrews beschreibt im Aust. J. Chem. 25 (1972) S. 639-646 die Hydrolyse von 2-Halo-6-nitrobenzonitril. Mit 38% Ausbeute ist die Hydrolyse des 2-Halo-6-nitrobenzonitrils unbefriedigend.

EP-A-529426 und DE-A-3409244 offenbaren die Oxidation von 2-Halo-6-nitrotoluol mit Salpetersäure. DE-A-3409244 beschreibt die Oxidation von 2-Halo-6-nitrotoluol mit verdünnter Salpetersäure bei 220°C und 30 bar. Die Umsetzung ist unvollständig, die Aufarbeitung kompliziert und der Reaktor wegen der erforderlichen Druckfestigkeit teuer. In EP-A-529426 wird eine drucklose Verfahrensweise zur Oxidation des 2-Halo-6-nitrotoluols mit Salpetersäure beschrieben. Die Reaktionsmischung ist nur 7,8%ig an Edukt, enthält zum größten Teil sehr korrosive 70%ige Schwefelsäure und muß bei Temperaturen von 175°C umgesetzt werden. Nach Extraktion mit o-Dichlorbenzol erhält man mit einer Ausbeute von circa 65% die gewünschte Säure in kristalliner Form.

Die Oxidation von 2-Halo-6-nitrobenzylalkoholen mit Kaliumpermanganat ist von Lehmstedt und Schrader in Chem.Ber. 70 (1937) S. 1526-1536 beschrieben. Dieses Vorgehen liefert befriedigende Ausbeuten, es wird jedoch als Zwangsanfallprodukt Braunstein gebildet, was für technische Realisierungen ungünstig ist. Außerdem ist Kaliumpermanganat kein preiswertes Oxidationsmittel. Lehmstedt und Schrader beschreiben außer der Oxidation des Alkohols auch dessen Präparation ausgehend von 2-Halo-6-nitrotoluolen, mit der Synthesefolge Bromierung, nukleophile Substitution zum Acetat und Verseifung. Dabei werden ebenfalls nur Konzentrationen an zu oxidierender Verbindung von circa 12% erreicht, was zu geringen Raumzeitausbeuten führt. Die wässrige Mischung aus der das Produkt durch Kristallisation gewonnen wird ist circa 7%ig an 2-Chlor-6-nitrobenzoesäure, die mit einer Ausbeute von 60% bezogen auf 2-Chlor-6-nitrotoluol gewonnen wird.

Gindraux beschreibt in Helv.Chim.Acta 12 (1929) S. 921-934 die Bromierung von 2-Halo-6-nitrotoluol als ca. 50%ige Lösung in o-Dichlorbenzol, die Hydrolyse des Benzylbromids mit Natriumcarbonat-Lösung und dessen Oxidation zum Aldehyd mit Bichromat/Schwefelsäure. Das chromhaltige Oxidationsmittel führt also nicht zur 2-Halo-6-nitrobenzoesäure und die entstehenden chromhaltigen Abfälle müssen wegen ihrer Toxizität aufwendig entsorgt werden.

2-Halo-6-nitrobenzoesäuren sind wichtige Zwischenprodukte, beispielsweise zur Herstellung von 6-Haloanthranilsäuren, die wiederum zur Herstellung von Pharmazeutika, Pflanzenschutz-Produkten und Farbstoffen dienen.

Aufgabe der vorliegenden Erfindung war es, ein Verfahren zu entwickeln, das 2-Halo-6-nitrobenzoesäuren hoher Reinheit mit guten Raumzeitausbeuten bei milden Reaktionsbedingungen zugänglich macht, so daß diese Verbindungen möglichst preiswert in großen Mengen produziert werden können.

Es wurde nun ein Verfahren zur Herstellung von 2-Halo-6-nitrobenzoesäuren der Formel (I), 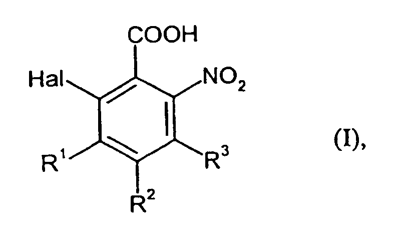
in der R¹, R² und R³ unabhängig voneinander Wasserstoff, Fluor, Chlor, Brom, Nitro oder Carboxyl bedeuten und Hal für Fluor, Chlor oder Brom steht, aus 2-Halo-6-nitrobenzylalkoholen, Estern oder Ethern der Formel (II) 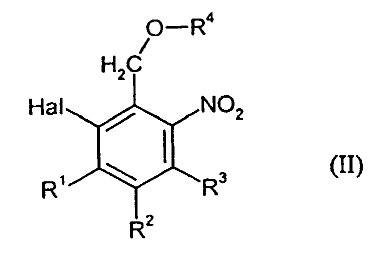 oder Mischungen dieser Verbindungen gefunden, wobei R¹, R², R³ und Hal die oben genannte Bedeutung haben und R⁴ für Wasserstoff, C₁-C₁₀ Alkyl, C₁-C₁₀ Carbonylalkyl oder 2-Halo-6-nitrobenzyl steht,
das dadurch gekennzeichnet ist, daß 2-Halo-6-nitrobenzylverbindungen der Formel (II) in Gegenwart von Salpetersäure auf Temperaturen zwischen 50 und 200°C erhitzt werden.

Ferner betrifft die Erfindung ein Verfahren zur Herstellung von 2-Halo-6-nitrobenzoesäuren der Formel (I) ausgehend von 2-Halo-6-nitrotoluolen der Formel (III) 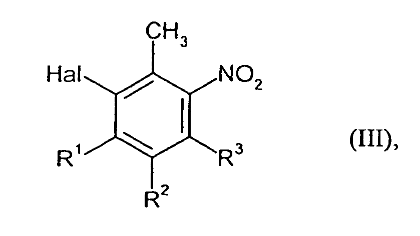 in der R¹, R², R³ und Hal die oben angegebene Bedeutung haben, das dadurch gekennzeichnet ist, daß ein 2-Halo-6-nitrotoluol der Formel (III) in das entsprechende 2-Halo-6-nitrobenzylbromid überführt wird, gefolgt von einer nukleophilen Substitution des Bromids zu den entsprechenden Verbindungen der Formel (II) und abschließender erfindungsgemäßer Salpetersäureoxidation.

Das erfindungsgemäße Verfahren erlaubt, 2-Halo-6-nitro-benzoesäuren mit guten Ausbeuten auf einfache Weise mit hoher Reinheit herzustellen. Wird von 2-Halo-6-nitrotoluolen ausgegangen, ist es nicht nötig, die Produkte der Bromierung oder der nukleophilen Substitution des Bromids in reiner Form zu isolieren, was eine sehr einfache Prozeßführung ermöglicht.

Bevorzugte 2-Halo-6-nitrobenzylverbindungen der Formel (II) sind solche, bei denen R¹, R², R³ und R⁴ für Wasserstoff und Häl für Fluor oder Chlor stehen. Als Ausgangsstoff besonders bevorzugt ist 2-Chlor-6-nitrobenzylalkohol.

Die Salpetersäure kann beispielsweise zwischen 10 und 95 Gew.-%, bevorzugt zwischen 35 und 90 Gew.-% Wasser enthalten. Es werden in der Regel zwischen 1 und 10 mol Salpetersäure pro mol Edukt eingesetzt. Bevorzugt ist die Verwendung von 2 bis 8 mol, besonders bevorzugt von 3 bis 5 mol Salpetersäure pro mol Edukt.

Die erfindungsgemäße Oxidation wird vorteilhaft bei Temperaturen zwischen 80°C und 180°C, bevorzugt zwischen 100 und 160°C, besonders bevorzugt zwischen 130 und 150°C durchgeführt.

Die Reaktionszeit kann beispielsweise 1 bis 30, bevorzugt 2 bis 15 Stunden betragen.

Die Verbindung der Formel (II) kann mit oder ohne Lösungsmittel der erfindungsgemäßen Oxidation unterworfen werden. Wird eine Verbindung der Formel (II) in Lösung eingesetzt, wird bevorzugt soviel Lösungsmittel zugesetzt , daß die organische Phase bis zu 75 Gew.-%, besonders bevorzugt bis zu 50 Gew.-% Lösungsmittel enthält.

Als Lösungsmittel geeignet sind beispielsweise alle schwer oxidierbaren Verbindungen mit Schmelzpunkten unterhalb 50°C, bevorzugt unterhalb 10°C und Siedepunkten höher als 100°C, bevorzugt höher als 150°C. Beispielsweise seien genannt: Nitrobenzol, 2-Chlor-nitrobenzol, 1,2-Dichlorbenzol, 1,3-Dichlorbenzol, 1,2,4-Trichlorbenzol, 1,2,3,4-Tetrachlorbenzol, 1,1,2,2-Tetrachlorethan, Pyridin, Pyrimidin, Benzonitril und Mischungen der genannten Lösungsmittel. Besonders bevorzugt wird 1,2-Dichlorbenzol eingesetzt.

In der Regel wird das erfindungsgemäße Verfahren bei Drucken zwischen 1 und 50 bar, bevorzugt bei 2 bis 15 bar durchgeführt. Es ist aber auch eine Reaktionsführung bei Normaldruck möglich.

In einer bevorzugten Ausführungsform wird die Verbindung der Formel (II) in der Art oxidiert, daß die Salpetersäure ganz oder teilweise vorgelegt und auf die gewünschte Reaktionstemperatur aufgeheizt wird. Dann wird die Verbindung der Formel (II) gegebenenfalls unter Dosierung weiterer Salpetersäure in 1 bis 20, bevorzugt in 2 bis 10 Stunden zudosiert. Dabei wird der Druck im Reaktionsgefäß nach einer kurzen Anfangsphase konstant gehalten. Nach erfolgter Zugabe wird 0,1 bis 10, bevorzugt 0,5 bis 2 Stunden nachgerührt.

Die Umsetzung erfolgt ganz besonders bevorzugt in Abwesenheit von Lösungsmitteln. Damit ist gemeint, daß Reste von Lösungsmitteln nicht mehr als 20 Gew.%, bevorzugt nicht mehr als 10 Gew.-%, besonders bevorzugt nicht mehr als 5 Gew.-% der zu oxidierenden Verbindung der Formel (II) beigemischt sind.

Gegebenenfalls können Oxidationskatalysatoren wie Chrom-, Molypdän-, Wolfram- oder Vanadiumverbindungen zugegen sein und außer Salpetersäure geringe Mengen Schwefelsäure. Bevorzugt wird ausschließlich mit Salpetersäure-Wasser-Mischungen oxidiert.

Die Oxidation kann auch kontinuierlich, z.B. in einem Verweilzeitrohrreaktor durchgeführt werden.

Erfindungsgemäß sind 2-Halo-6-nitrobenzoesäuren der Formel (I) auch in einem mehrstufigen Verfahren ausgehend von 2-Halo-6-nitrotoluolen der Formel (III) zugänglich. Dazu wird zunächst auf an sich bekannte Weise bromiert, dann auf an sich bekannte Weise das erhaltene Bromid nukleophil zu den entsprechenden Verbindungen der Formel (II) substituiert und abschließend erfindungsgemäß mit Salpetersäure oxidiert.

Bevorzugte Verbindungen der Formel (III) sind solche, bei denen R¹, R² und R³ für Wasserstoff und Hal für Fluor oder Chlor stehen. Besonders bevorzugt ist 2-Chlor-6-nitrotoluol.

Bromiert wird bevorzugt mit elementarem Brom oder N-Brom-succinimid.

Die nukleophile Substitution des Bromids erfolgt vorzugsweise durch Umsetzung mit Alkali- oder Erdalkalicarbonaten und/oder -hydrogencarbonaten, durch Umsetzung mit organischen Alkali- oder Erdalkalicarboxylaten oder durch Umsetzung mit Alkali- oder Erdalkalialkoholaten oder -hydroxyden.

Bevorzugt werden die ersten beiden Reaktionsschritte (Bromierung, Substitution des Bromids) in Gegenwart eines Lösungsmittels durchgeführt. Es können die Lösungsmittel eingesetzt werden, die bereits oben beschrieben sind. Besonders bevorzugt ist 1,2-Dichlorbenzol.

Bevorzugt werden diese Verfahrensschritte ohne Zwischenisolierung des Bromids und ohne Zwischenisolierung des Alkohols, Ethers oder Esters und ohne Lösungsmittelwechsel durchgeführt und die erfindungsgemäße Oxidation unmittelbar angeschlossen. Am Ende der Reaktionsfolge kann die hergestellte Säure der Formel (I) in hoher Reinheit durch Kristallisation gewonnen werden.

Besonders bevorzugt wird nach der Stufe der Bromidsubstitution das Lösungsmittel durch Verdampfung ganz oder teilweise von der Verbindung der Formel (II) abgetrennt.

Die Kombination der Verfahrensschritte Bromierung, Bromidsubstitution und Oxidation mit Salpetersäure ermöglicht es, ausgehend von 2-Halo-6-nitrotoluolen der Formel (III) 2-Halo-6-nitrobenzoesäuren der Formel (I) über eine technisch sehr einfach zu realisierende Reaktionsfolge herzustellen.

Das erfindungsgemäße Verfahren kann beispielsweise wie folgt durchgeführt werden. In einem Kessel mit Rührer, Einleitungsrohr und Kondensator wird ein Lösungsmittel vorgelegt und ein 2-Halo-6-nitrotoluol der Formel (III) gelöst. Bei Temperaturen zwischen 120 und 200°C, bevorzugt zwischen 140 und 180°C wird elementares Brom über das Rohr eingeleitet. Nach dem Beenden der Bromierung wird auf 20 bis 50°C abgekühlt und mit Stickstoff gelöstes HBr und Reste Brom ausgetrieben. Anschließend wird eine wäßrige Natriumcarbonatlösung zugepumpt und die Reaktionsmischung unter Eigendruck auf 80 bis 160°C, bevorzugt auf 90 bis 130°C aufgeheizt. Nach Beendigung der Bromsubstitution kühlt man auf 20 bis 80°C ab und trennt die wäßrige Phase von der organischen. Zur organischen Phase wird bei 80 bis 160°C, bevorzugt 90 bis 140°C 65 Gew.%ige wässrige Salpetersäure gepumpt und bis zur vollständigen Oxidation gerührt. Dabei kann entstehendes Wasser abdestilliert werden, so daß die Säurekonzentration konstant bleibt. Nach Beendigung der Oxidation wird die Salpetersäure abdestilliert, so daß nur organische Phase im Kessel übrig bleibt und die 2-Halo-6-nitrobenzoesäure der Formel (I) durch Abkühlen der Lösung auf etwa 25 °C kristallisiert.

Die Kristallisation wird durch weiteres Kühlen auf etwa 0°C vervollständigt und die Kristalle durch Filtration abgetrennt. Nachwaschen mit wenig reinem kalten Lösungsmittel liefert reine 2-Halo-6-nitrobenzoesäure der Formel (I) mit Ausbeuten, die im Allgemeinen zwischen 50% und 80% bezogen auf das eingesetzte 2-Halo-6-nitrotoluol der Formel (III) liegen.

Bevorzugt wird die Oxidation des Alkohols invers durchgeführt, d.h. die Salpetersäure wird vorgelegt und der Alkohol hinzu gepumpt. Vorteilhaft wird der Alkohol vor dem Zugeben von Lösungsmittel befreit.

Durch die inverse Reaktionsführung können bei der erfindungsgemäßen Oxidation Ausbeuten bis zu 95% der Theorie erhalten werden. Die inverse Reaktionsführung läßt sich zudem im technischen Maßstab sehr sicher durchführen.

Besonders bewährt hat sich das erfindungsgemäße Verfahren zur Herstellung von 2-Chlor-6-nitrobenzoesäure.

Das erfindungsgemäße Verfahren zur Herstellung von 2-Halo-6-nitrobenzoesäuren wird anhand der folgenden Beispiele weiter erläutert, ohne daß es in irgendeiner Weise auf diese Beispiele beschränkt wäre.

### Beispiele

Soweit nicht anders angegeben, handelt es sich bei Prozentangaben um Angaben in mol%. Ausbeuteangaben sind bezogen auf eingesetztes 2-Chlor-6-nitro-toluol.

### Beispiel 1

### Bromierung von 2-Chlor-6-nitrotoluol

400 g 2-Chlor-6-nitrotoluol wurden in 369 g 1,2-Dichlorbenzol gelöst und unter Stickstoff auf 165°C aufgeheizt. Nach Erreichen der Reaktionstemperatur wurden 447 g Brom innerhalb von 6 Stunden zudosiert und eine halbe Stunde nachgerührt. Der entstehende HBr-Strom wurde durch einen Kühler geführt, um mitgerissenes Brom niederzuschlagen und der Reaktion wieder zuzuführen.

Nach erfolgter Bromierung wurde auf 100°C abgekühlt und HBr und Bromreste mit Stickstoff ausgetrieben.

Die Analyse der Mischung wies 0,47 mol% unumgesetztes 2-Chlor-6-nitrotoluol und 99,4 mol% 2-Chlor-6-nitrobenzylbromid aus.

### Beispiel 2

### Herstellung von 2-Chlor-6-nitrobenzylalkohol

Die Reaktionsmischung aus Beispiel 1 wurde auf 40°C abgekühlt und mit 120mol% (bezogen auf eingesetzes 2-Chlor-6-nitrotoluol) einer 20 Gew.-%igen wässrigen Natriumcarbonatlösung versetzt. Die gut gerührte Mischung wurde auf 120°C aufgeheizt und 6 Stunden bei dieser Temperatur gerührt, wobei ein Druck von 5-6 bar entstand.

Das Reaktionsgemisch wurde auf 40°C abgekühlt und die wäßrige von der organischen Phase getrennt.

Die Analyse der organischen Phase wies 0,6 mol% unumgesetztes 2-Chlor-6-nitrotoluol, 0,1 mol% unumgesetztes 2-Chlor-6-nitrobenzylbromid und 98 mol% 2-Chlor-6-nitrobenzylalkohol aus.

### Beispiel 3

### Oxidation von 2-Chlor-6-nitrobenzylalkohol

Die organische Phase aus Beispiel 2 wurde auf 140°C aufgeheizt und binnen 4 Stunden mit 220 mol% (bezogen auf eingesetztes 2-Chlor-6-nitrotoluol) 65 Gew.-%iger Salpetersäure versetzt und 2 Stunden nachgerührt. Dabei destillierte eine ca. 9 Gew.-%ige wässrige Salpetersäure ab und Nitrose Gase entwichen. Mitdestilliertes 1,2-Dichlorbenzol wurde dem Ansatz wieder zugeführt.

Restliche Salpetersäure-Wasser-Mischung wurde abdestilliert und die Reaktionsmischung binnen 4 Stunden auf 0°C abgekühlt. Die ausgefallene 2-Chlor-6-nitrobenzoesäure wurde abfiltriert und mit wenig kaltem 1,2-Dichlorbenzol gewaschen.

Nach dem Trocknen erhielt man in 60%iger Ausbeute (bezogen auf 2-Chlor-6-nitrotoluol) reine 2-Chlor-6-nitrobenzoesäure.

### Beispiel 4

### Inverse Oxidation von 2-Chlor-6-nitrobenzylalkohol

Die organische Phase aus Beispiel 2 wurde auf 100°C aufgeheizt und bei 15 mbar in 2 Stunden 1,2-Dichlorbenzol bis auf einen Restgehalt von 4 Gew.-% abdestilliert.

400 mol% (bezogen auf eingesetztes 2-Chlor-6-nitrotoluol) 65 Gew.-%ige Salpetersäure wurden in einem Autoklaven vorgelegt und auf 140°C aufgeheizt. Der vom Lösungsmittel weitgehend befreite Benzylalkohol wurde gleichmäßig binnen 8,5 Stunden zudosiert. Dabei wurde der Druck im Autoklaven bei 10 bar konstant gehalten. Entweichende Salpetersäure und 1,2-Dichlorbenzol wurde nicht ersetzt. Nachdem die gesamte Menge Benzylalkohol zugepumpt worden ist wurde 10 Minuten nachgerührt, auf 100°C abgekühlt und entspannt.

Wässrige Salpetersäure wurde bei leichtem Vakuum abdestilliert und der verbleibende Rückstand mit 400 g 1,2-Dichlorbenzol gelöst.

Die Analyse der Mischung wies 1,44 mol% unumgesetztes 2-Chlor-6-nitrobenzylalkohol, 0,26 mol% 2-Chlor-6-nitrobenzaldehyd und 90 mol% 2-Chlor-6-nitrobenzoesäure aus.

Das Gemisch wurde binnen 4 Stunden auf 25°C abgekühlt. Die ausgefallene 2-Chlor-6-nitrobenzoesäure wurde abfiltriert und mit wenig kaltem 1,2-Dichlorbenzol gewaschen. Nach dem Trocknen erhielt man in 78%iger Ausbeute (bezogen auf eingesetztes 2-Chlor-6-nitrotoluol) reine 2-Chlor-6-nitrobenzoesäure.

## Patentansprüche

1. Verfahren zur Herstellung von 2-Halo-6-nitrobenzoesäuren der Formel (I), 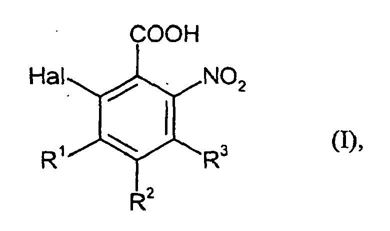
in der R¹, R² und R³ unabhängig voneinander Wasserstoff, Fluor, Chlor, Brom, Nitro oder Carboxyl bedeuten und Hal für Fluor, Chlor oder Brom steht,
aus 2-Halo-6-nitrobenzylalkoholen, Estern oder Ethern der Formel (II) 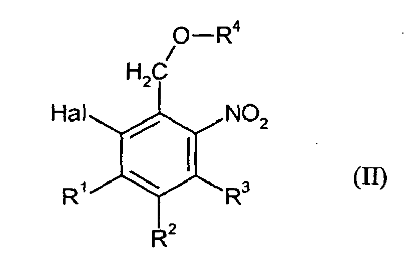
oder Mischungen dieser Verbindungen, wobei R¹, R², R³ und Hal die oben genannte Bedeutung haben und R⁴ für Wasserstoff, C₁-C₁₀ Alkyl, C₁-C₁₀ Carbonylalkyl oder 2-Halo-6-nitrobenzyl steht,
**dadurch gekennzeichnet, daß** 2-Halo-6-nitrobenzylverbindungen der Formel (II) in Gegenwart von Salpetersäure auf Temperaturen zwischen 50 und 200°C erhitzt werden.

2. Verfahren nach Anspruch 1, **dadurch gekennzeichnet, daß** R¹, R², R³ und R⁴ für Wasserstoff und Hal für Fluor oder Chlor stehen.

3. Verfahren nach wenigstens einem der Ansprüche 1 bis 2, **dadurch gekennzeichnet, daß** 2-Chlor-6-nitrobenzylalkohol eingesetzt wird.

4. Verfahren nach wenigstens einem der Ansprüche 1 bis 3, **dadurch gekennzeichnet, daß** 2 bis 8 mol Salpetersäure pro mol Edukt eingesetzt werden.

5. Verfahren nach wenigstens einem der Ansprüche 1 bis 4, **dadurch gekennzeichnet, daß** die Salpetersäure ganz oder teilweise vorgelegt und die zu oxidierende 2-Halo-6-nitrobenzylverbindung der Formel (II) zudosiert wird.

6. Verfahren nach Anspruch 5, **dadurch gekennzeichnet, daß** der zu oxidierenden Verbindung nicht mehr als 20 Gew.-% eines Lösungsmittels, bezogen auf die zu oxidierende Verbindung, beigemischt sind.

7. Verfahren nach wenigstens einem der Ansprüche 1 bis 6 **dadurch gekennzeichnet, dass** die 2-Halo-6-nitrobenzylalkohole, Ester oder Ether der Formel (II) 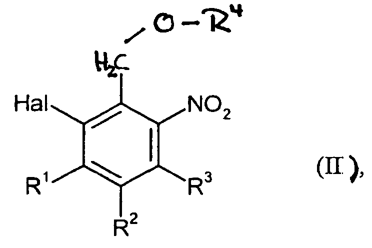
in der Hal, R¹, R², R³ und R⁴ die in Anspruch 1 genannte Bedeutung haben,
hergestellt werden, indem ein 2-Halo-6-nitrotoluol der Formel (III) 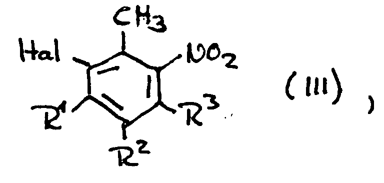 in der Hal, R¹, R² und R³ die in Anspruch genannte Bedeutung Saben, in das entsprechende 2-Halo-6-nitrobenzylbromid überführt wird, gefolgt von einer nukleophilen Substitution des Bromids.

8. Verfahren nach Anspruch 7, **dadurch gekennzeichnet, daß** ein 2-Halo-6-nitrotoluol der Formel (III) eingesetzt wird, wobei R¹, R² und R³ für Wasserstoff stehen und Hal Fluor oder Chlor bedeutet.

9. Verfahren nach mindestens einem der Ansprüche 7 bis 8, **dadurch gekennzeichnet, daß** die Verfahrensschritte Bromierung und nukleophile Substitution des Bromids ohne Isolierung der entstehenden Produkte durchgeführt werden.

10. Verfahren nach mindestens einem der Ansprüche 7 bis 9, **dadurch gekennzeichnet, daß** die Verfahrensschritte Bromierung, und nukleophile Substitution des Bromids in Gegenwart eines Lösungsmittels ohne einen Lösungsmittelwechsel durchgeführt werden, dann das Lösungsmittel so weit entfernt wird, daß bezogen auf die zu oxidierende Verbindung nicht mehr als 20 Gew.-% Lösungsmittel vorliegen, und abschließend oxidiert wird.

## Claims

1. Process for the preparation of 2-halo-6-nitrobenzoic acids of the formula (I) 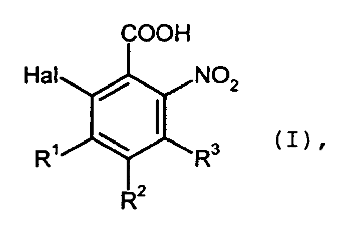
in which R¹, R² and R³, independently of one another, are hydrogen, fluorine, chlorine, bromine, nitro or carboxyl, and Hal is fluorine, chlorine or bromine,
from 2-halo-6-nitrobenzyl alcohols, esters or ethers of the formula (II) 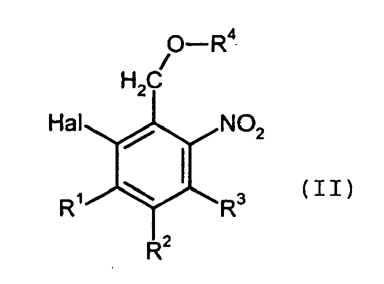
or mixtures of these compounds, where R¹, R², R³ and Hal have the meanings given above, and R⁴ is hydrogen, C₁-C₁₀-alkyl, C₁-C₁₀-carbonylalkyl or 2-halo-6-nitrobenzyl,
**characterized in that** 2-halo-6-nitrobenzyl compounds of the formula (II) are heated in the presence of nitric acid to temperatures between 50 and 200°C.

2. Process according to Claim 1, **characterized in that** R¹, R², R³ and R⁴ are hydrogen, and Hal is fluorine or chlorine.

3. Process according to at least one of Claims 1 to 2, **characterized in that** 2-chloro-6-nitrobenzyl alcohol is used.

4. Process according to at least one of Claims 1 to 3, **characterized in that** 2 to 8 mol of nitric acid are used per mole of starting material.

5. Process according to at least one of Claims 1 to 4, **characterized in that** some or all of the nitric acid is initially introduced and the 2-halo-6-nitrobenzyl compound of the formula (II) to be oxidized is metered in.

6. Process according to Claim 5, **characterized in that** not more than 20% by weight of a solvent, based on the compound to be oxidized, are admixed with the compound to be oxidized.

7. Process according to at least one of Claims 1 to 6,
**characterized in that** the 2-halo-6-nitrobenzyl alcohols, esters or ethers of the formula (II) 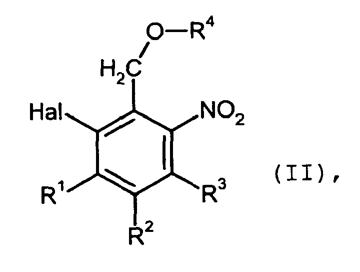
in which Hal, R¹, R², R³, and R⁴ have the meanings given in Claim 1,
are prepared by converting a 2-halo-6-nitrotoluene of the formula (III) 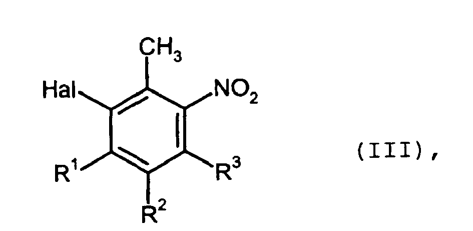
in which Hal, R¹, R² and R³ have the meanings given in Claim 1,
into the corresponding 2-halo-6-nitrobenzyl bromide, followed by a nucleophilic substitution of the bromide.

8. Process according to Claim 7, **characterized in that** a 2-halo-6-nitrotoluene of the formula (III) is used, where R¹, R² and R³ are hydrogen, and Hal is fluorine or chlorine.

9. Process according to at least one of Claims 7 to 8, **characterized in that** the process steps bromination and nucleophilic substitution of the bromide are carried out without isolation of the resulting products.

10. Process according to at least one of Claims 7 to 9, **characterized in that** the process steps bromination and nucleophilic substitution of the bromide are carried out in the presence of a solvent or without a solvent change, then the solvent is removed such that, based on the compound to be oxidized, not more than 20% by weight of solvent are present, and, finally, oxidation is carried out.

## Revendications

1. Procédé de préparation d'acides 2-halogéno-6-nitrobenzoïques de formule (I), 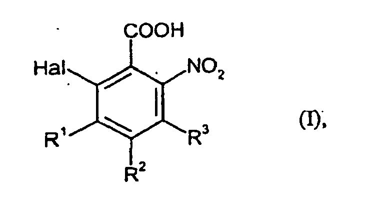
dans laquelle R¹, R² et R³, indépendamment les uns des autres, représentent un hydrogène, un fluor, un chlore, un brome, un nitro ou un carboxyle et Hal représente un fluor, un chlore ou un brome,
à partir d'alcools, d'esters ou d'éthers 2-halogéno-6-nitrobenzyliques de formule (II) 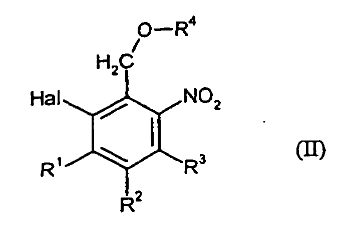
ou de mélanges de ces composés, dans laquelle R¹, R², R³ et Hal présentent la signification susmentionnée et R⁴ représente un hydrogène, un alkyle en C₁-C₁₀, un C₁-C₁₀-carbonylalkyle ou un 2-halogéno-6-nitrobenzyle,
**caractérisé en ce que** les composés 2-halogéno-6-nitrobenzyliques de formule (II) sont chauffés en présence d'acide nitrique à des températures comprises entre 50 et 200°C.

2. Procédé selon la revendication 1, **caractérisé en ce que** R¹, R², R³ et R⁴ représentent un hydrogène et Hal représente un fluor ou un chlore.

3. Procédé selon au moins l'une des revendications 1 à 2, **caractérisé en ce que** l'on utilise l'alcool 2-chloro-6-nitrobenzylique.

4. Procédé selon au moins l'une des revendications 1 à 3, **caractérisé en ce que** l'on utilise de 2 à 8 mol d'acide nitrique par mole de substance de départ.

5. Procédé selon au moins l'une des revendications 1 à 4, **caractérisé en ce que** l'acide nitrique est entièrement ou partiellement introduit et le composé 2-halogéno-6-nitrobenzylique à oxyder de formule (II) est ajouté en quantité mesurée.

6. Procédé selon la revendication 5, **caractérisé en ce que** pas plus de 20 % en poids d'un solvant, par rapport au composé à oxyder, sont ajoutés au composé à oxyder.

7. Procédé selon au moins l'une des revendications 1 à 6, **caractérisé en ce que** les alcools, esters ou éthers 2-halogéno-6-nitrobenzyliques de formule (II) 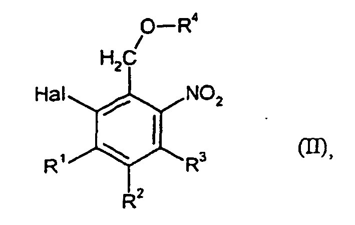
dans laquelle Hal, R¹, R², R³ et R⁴ présentent la signification mentionnée dans la revendication 1,
sont préparés en transformant un 2-halogéno-6-nitrotoluène de formule (III), 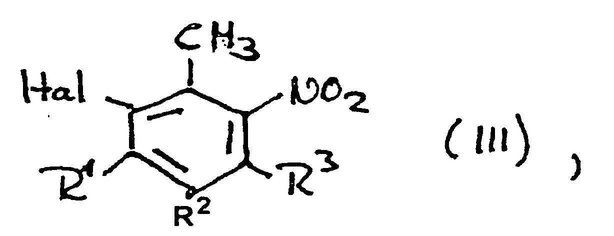
dans laquelle Hal, R¹, R² et R³ présentent la signification mentionnée dans la revendication 1, en le bromure de 2-halogéno-6-nitrobenzyle correspondant, suivi d'une substitution nucléophile du bromure.

8. Procédé selon la revendication 7, **caractérisé en ce que** l'on utilise un 2-halogéno-6-nitrotoluène de formule (III) dans laquelle R¹, R² et R³ représentent un hydrogène et Hal représente un fluor ou un chlore.

9. Procédé selon au moins l'une des revendications 7 à 8, **caractérisé en ce que** les étapes de procédé de bromation et de substitution nucléophile du bromure sont réalisées sans isolation des produits formés.

10. Procédé selon au moins l'une des revendications 7 à 9, **caractérisé en ce que** les étapes de procédé de bromation et de substitution nucléophile du bromure sont réalisées en présence d'un solvant sans changement de solvant, puis le solvant est éliminé jusqu'à ce que, par rapport au composé à oxyder, pas plus de 20 % en poids de solvant soient présents, et on procède enfin à l'oxydation.
